# EUROPÄISCHE PATENTANMELDUNG

(11) **EP 0 652 432 A1**
(43) Veröffentlichungstag der Anmeldung: **10.05.1995**
(21) Anmeldenummer: 93117852.9
(22) Anmeldetag: 04.11.1993
(51) Int. Cl.: G01N 21/89, G01N 33/36, D06H 3/08

(54) **Vorrichtung zum Erkennen von Fremdmaterial, insbesondere von Fremdfasern, in einem längsbewegten textilen Gebilde**

(71) Anmelder: BARCO nv/Automation, B-8500 Kortrijk (BE); W. SCHLAFHORST AG & CO., D-41061 Mönchengladbach (DE)
(72) Erfinder: Remmerie, Johan, B-8500 Kortrijk (BE); Bouvyn, Patrick, B-8790 Waregem (BE)
(74) Vertreter: Hamann, Arndt, Dipl.-Ing.

(57) **Zusammenfassung**

Die Erfindung betrifft eine Detektoreinrichtung (1) zum Erkennen von Fremdmaterial in einem längsbewegten textilen Gebilde (13). Die Detektoreinrichtung (1) besteht im wesentlichen aus einer in einem Beleuchtungsteil (3) angeordneten polychromaten Lichtquelle (6), einer in einem Empfangsteil (4) angeordneten Sensoreinrichtung (7) und einem Fadenabtastraum (5) mit einem Spiegelelement (14).

Die erfindungsgemäße geometrische Anordnung dieser Komponenten sowie die Verwendung eines Wellenlängen - selektiven Photoempfängers mit vertikal gestaffelt angeordneten Photodioden (16, 17) führen zu einer Fremdfasererkennungsvorrichtung, die unempfindlich gegen Garndurchmesserschankungen und Garnstrukturänderungen sowie unabhängig bezüglich der Garnfarbe des zu überprüfenden Fadens (13) ist.

## Beschreibung

Die Erfindung betrifft eine Vorrichtung zum Erkennen von Fremdmaterial, insbesondere von Fremdfasern, in einem längsbewegten textilen Gebilde, mit einer Detektoreinrichtung, die wenigstens eine Licht emittierende Lichtquelle sowie eine Sensoreinrichtung zum Empfangen von reflektiertem Licht aufweist.

Es ist bekannt, daß in einen Faden eingesponnene Fremdfasern in der Regel eine Farbdifferenz hervorrufen, die durch Messen der "Farbe" des Garnes erkannt werden kann. Mit Farbe wird dabei die Spektralverteilung des Lichtes bezeichnet, das, von einem Objekt reflektiert, vom menschlichen Auge wahrnehmbar ist. Alle bekannten Versuche der Fremdfasererkennung im Garn beginnen daher mit der Annahme, daß sich die Reflexion von Fremdmaterial von der des Garnes unterscheidet.

Das britische Patent 2 095 828 B beschreibt eine Fremdfasererkennungseinrichtung, die mit einer Sensoreinrichtung arbeitet, die das textile Gebilde an beabstandeten Stellen abtastet.

Eine erste Sensoreinrichtung erfaßt dabei das durch eine Stoffbahn transmittierte Licht einer auf der entgegengesetzten Stoffbahnseite angeordneten Lichtquelle. Eine zweite Sensoreinrichtung, die in Laufrichtung der Stoffbahn in einem vorbestimmten Abstand hinter der ersten Sensoreinrichtung angeordnet ist und von dieser über eine Abschirmung optisch getrennt ist, wird mit Licht beaufschlagt, das zwei oberhalb der Stoffbahn angeordnete Lichtquellen emittieren und das von der Stoffbahn teilweise reflektiert wird. Im Bereich dieser zweiten Sensoreinrichtung ist auf der entgegengesetzten Stoffbahnseite zusätzlich ein Streulichtreflektor angeordnet, dessen Farbe an die Farbe der Stoffbahn angepaßt ist.

Die Sensoreinrichtungen und der Antrieb für die Stoffbahn sind über eine Steuereinrichtung so aufeinander abgestimmt, daß die Sensoreinrichtungen mit einer zeitlichen Verzögerung dieselben Stoffbahnbereiche überprüfen. Die Fremdfasererkennungseinrichtung überprüft dabei die Stoffbahnen hinsichtlich ihres Transmissions- sowie ihres Reflexionsvermögens und nimmt durch Vergleich mit vorgegebenen SOLL-Werten eine Klassifizierung der auftretenden Fehler vor.

Da die Meßwerte, aus denen auf die Anwesenheit Zum Beispiel einer Fremdfaser geschlossen wird, an beabstandeten Meßpunkten genommen werden, sind Fehlinterpretationen, beispielsweise infolge unkorrekter Laufgeschwindigkeit der Stoffbahn, nicht auszuschließen.

Das beschriebene Verfahren mit einer Streulichtbeleuchtung, bei der das Licht von allen Seiten auf das Garn scheint und einem Hintergrund, der vom Sensor direkt einsehbar ist, ist im Prinzip mit einem schwarz-weiß Bild vergleichbar, bei dem ein Garn vor einem Hintergrund in dergleichen Farbe liegt. Die Sensoreinrichtung erfaßt den gesamten Weißinhalt des Bildes "Garn-Hintergrund". Dieses Weißniveau wird durch jede im Garn vorhandene, mit dem Basismaterial kontrastierende Fremdfaser beeinflußt. Nachteilig bei diesem Verfahren ist vor allem, daß, wenn sich der Weißgehalt oder eine andere Hauptfarbe des Hintergrundes von der Farbe des Garnes unterscheidet, jede Dick- oder Dünnstelle im Garn dazu führt, daß weniger oder mehr vom Hintergrund sichtbar wird und damit jede Garndurchmesserschwankung oder Garnstrukturänderung als scheinbare Anwesenheit einer Fremdfaser detektiert wird.

Die Einrichtung gemäß GB-PS 2 095 828 B hat daher insbesondere den Nachteil, daß es zwingend erforderlich ist, die Farbe des Hintergrundes jeweils an die Farbe des zu untersuchenden textilen Gebildes anzupassen.

Die durch die PCT-Anmeldung WO 93/13 407 bekannte Fremdfasererfassungsvorrichtung vermeidet solche durch Garndurchmesserschwankungen verursachten Fehlinterpretationen durch eine aufwendige optische Einrichtung.

Die dargestellte und beschriebene Vorrichtung weist in einem sogenannten Quellenteil ihres Gehäuses eine Lichtquelle, beispielsweise eine Leuchtdiode, einen Lichtzuführer, der das Licht in zwei Strahlenbündel aufteilt, zwei Eingangsprismen, eine erstes Austrittsprisma, einen zweiten Lichtzuführer sowie einen ersten Sensor auf. Im Sensorteil des Gehäuses sind außerdem ein zweites Austrittsprisma, ein dritter Lichtzuführer sowie ein zweiter Sensor angeordnet.

Dieser recht aufwendige optische Teil ist mit einer elektronischen Auswerteeinheit verbunden, so daß es mit Hilfe des Garnes möglich ist, die reflektierten und die transmittierten Lichtanteile zu erfassen. Bei einer Dickstelle im Garn wächst der Anteil der Reflexion, der Anteil der Transmission verringert sich entsprechend. Die Summe der Signale wird mittels der elektronischen Auswerteeinheit konstant gehalten. Für eine vorgegebene Garnfarbe wird ein Signal entsprechend verstärkt und damit ein künstlicher Hintergrund in der Farbe des Garnes geschaffen, wobei die Intensität des Hintergrundes direkt gemessen und nachgeregelt wird bis dessen Weißgehalt an dem Weißgehalt des Garnes angepaßt ist.

Insbesondere das Erfassen der Reflexion und der Transmission physikalisch an derselben Stelle des Garnes führt zu einer, wie vorstehend beschrieben, äußerst aufwendigen und entsprechend kostenintensiven optischen Einrichtung.

Die vorbeschriebenen Einrichtungen arbeiten bei der Erfassung von Farbdifferenzen im Garn mit einer Wellenlänge, das heißt, sie "sehen" das Garn schwarz-weiß.

In der WO 93/13 407 ist zwar bereits grundsätzlich auf die Möglichkeit einer Echtfarbmessung durch den Einsatz von Sensoreinrichtungen hingewiesen, die verschiedene Wellenlängen des Lichtes erfassen, die entsprechenden Ausführungsbeispiele sind jedoch entweder nicht praktikabel oder technisch äußerst aufwendig.

Es wird beispielsweise vorgeschlagen, für die Messung der Reflexion und für die Messung der Transmission jeweils einen eigenen Sensor einzusetzen, wobei diese Sensoren auf unterschiedlichen Wellenlängen arbeiten. Eine solche Anordnung ist technisch sinnlos, da sie lediglich zu einer unterschiedlichen Verstärkung vor einem künstlichen Hintergrund führt.

Ein weiterer Vorschlag sieht die Verwendung mehrerer Lichtquellen vor, die nacheinander Licht mit verschiedenen Wellenlängen emittieren. Die in diesem Zusammenhang einzusetzenden Lichtsensoren sind dabei auf sämtlichen Wellenlängen des emittierten Lichtes empfindlich.

Eine solche Anordnung ist theoretisch zwar möglich, in der Praxis jedoch sehr kompliziert und aufwendig.

Der Erfindung liegt, ausgehend vom vorbeschriebenen Stand der Technik, die Aufgabe zugrunde, eine Vorrichtung zu schaffen, die, unter Vermeidung der Nachteile der bekannten Einrichtungen eine zuverlässige Erkennung von in textilen Gebilden befindlichen Fremdmaterialien, insbesondere von Fremdfasern, ermöglicht und dabei kostengünstig gefertigt werden kann.

Diese Aufgabe wird erfindungsgemäß durch eine Vorrichtung gelöst, wie sie im Anspruch 1 beschrieben ist.

Die erfindungsgemäße Ausführungsform hat dabei insbesondere den Vorteil, daß das reflektierte Licht auf wenigstens zwei Wellenlängen gleichzeitig erfaßt wird. Die gewählte Anordnung der polychromaten Lichtquelle und des Photoempfängers, mit wenigstens zwei vertikal gestaffelt angeordneten Photodetektoren stellt dabei sicher, daß der optische Weg des emittierten und reflektierten Lichtes für alle Wellenlängen identisch ist und damit die empfangenen, reflektierten Lichtkomponenten exakt von der gleichen Stelle des Garnes stammen.

In weiterer Ausgestaltung der Erfindung sind Mittel zur Abschattung der Photodetektoren gegenüber solchen Lichtkomponenten vorgesehen, die nicht der direkten Garnreflexion entstammen. Das heißt, diese Abschattungsmittel sorgen dafür, daß eventuelle Hintergrundreflexionen oder Streulichtreflexionen für die Photodetektoren nicht erfaßbar sind.

In bevorzugter Ausführungsform ist als Abschattungsmittel ein Spiegelelement vorgesehen, das entsprechend der pysikalischen Gesetzmäßigkeit Lichteinfallswinkel = Lichtausfallswinkel so positioniert wird, daß Hintergrundreflexion und/oder Streulichtreflexion für die Photodetektoren unsichtbar bleibt.

Das bedeutet, es findet eine optische Kontrolle des Weges der Lichtstrahlen statt, die nicht vom Garn reflektiert werden.

In weiterer Ausgestaltung der Erfindung ist eine, die Signale der vertikal gestaffelt angeordneten Photodioden verarbeitende Steuereinrichtung vorgesehen. Vorteilhafterweise wird dabei in einem Komparator das Verhältnis der Meßströme gebildet, die an den Photodioden aufgrund der polychromaten Lichtquelle entstehen. Das Verhältnis der Meßströme entspricht dabei der Differenz der Logarithmen, wobei als Logarithmusfunktion die Logarithmuscharakteristik eines Transistors (Basis-Emitter-Diode) Anwendung findet. Da sich bei Garndurchmesserschwankungen oder Garnstrukturänderungen auf allen Wellenlängen zwar die Intensität des Lichtes nicht aber dessen spektrale Zusammensetzung, sprich Farbe, ändert, werden auf diese Weise Einflüsse, die von Garndurchmesserschwankungen, Garnstrukturänderungen oder dergleichen herrühren, ausgeschaltet, so daß das ermittelte Meßergebnis eine reine Farbinformation enthält.

In vorteilhafter Ausführungsform ist die Lichtquelle in einem Beleuchtungsteil der Detektoreinrichtung angeordnet und von der in einem Empfangsteil angeordneten Sensoreinrichtung durch eine lichtdichte Abschirmung optisch getrennt. Lediglich im Bodenbereich sowohl des Beleuchtungs- als auch des Empfangsteils sind jeweils eine Lichtaustritts- beziehungsweise eine Lichteintrittsöffnung vorgesehen. Im Abstand vor diesen Öffnungen ist ein Spiegelelement angeordnet. Die geometrische Zuordnung der vorgenannten Mittel ist dabei so getroffen, daß das von der Lichtquelle emittierte, durch die Lichtaustrittsöffnung austretende Licht einen definierten Bereich ausleuchtet, der in Verbindung mit einem vom Photoempfänger erfaßbaren Bereich einen sogenannten Meßbereich bildet. Die Lichtquelle beleuchtet dabei den Meßbereich, der von einem zu überwachenden Faden gekreuzt wird, aus einer Richtung. Die einheitliche spektrale Zusammensetzung des Lichtes innerhalb des Meßbereiches gewährleistet, daß jeder Punkt des Meßbereiches mit Licht in der gleichen Farbe ausgeleuchtet wird. Außerdem ist jeder Punkt des Meßbereiches von den gestaffelt angeordneten Photodioden des Sensorelementes unter einem identischen Winkel einsehbar.

In weiterer Ausgestaltung der Erfindung ist das Spiegelelement innerhalb des Meßbereiches in einem solchen Abstand vor den Lichtaustritts- beziehungsweise Lichteintrittöffnungen angeordnet, daß unter Berücksichtigung der physikalischen Gesetzmäßigkeit Lichteinfallswinkel = Lichtausfallswinkel gewährleistet ist, daß der Anteil des von der Lichtquelle emittierten Lichtes, der nicht direkt von dem den Meßbereich querenden Garn reflektiert wird, für das Sensorelement unsichtbar bleibt. Das heißt, es wird verhindert, daß dieser Lichtanteil in die Lichteintrittsöffnung reflektiert wird. Der durch eine solche Anordnung entstehende "schwarze Spiegel" bildet einen künstlichen Horizont, der für das Sensorelement unsichtbar ist.

Da bei verschiedenen, zum Beispiel abriebsempfindlichen Garnen nicht ausgeschlossen werden kann, daß das Spiegelelement durch Staub oder dergleichen verschmutzt wird, was zu einer diffusen Reflexion des von der Lichtquelle emittierten Lichtes führen kann, ist gemäß einer weiteren vorteilhaften Ausführungsform vorgesehen, den Spiegel relativ weit von den vorbeschriebenen Lichtöffnungen entfernt, beispielsweise am unteren Ende des Meßbereiches anzuordnen. Durch eine solche Anordnung können Einflüsse, die durch Streulicht entstehen, weitestgehend vermieden werden.

Die vorbeschriebenen Maßnahmen haben insbesondere den Vorteil, daß aufgrund der "Hintergrundentfernung" sowohl Garndurchmesserschwankungen oder Garnstrukturänderungen als auch die Farbe des zu überprüfenden Gebildes ohne Einfluß auf das Meßergebnis bleiben. Bezüglich der Basisfarbe des zu überwachenden Garnes bestehen daher keine Einschränkungen.

In vorteilhafter Weiterbildung der Erfindung findet eine polychromate Lichtquelle Verwendung, deren emittiertes Licht ein Spektrum aufweist, das sich vom sichtbaren - bis in den nahen Infrarotbereich erstreckt. Das Licht weist dabei wenigstens auf Zwei Wellenlängen eine von den vertikal gestaffelt angeordneten Photodioden verwertbare Intensität auf. Außerdem ist dafür Sorge getragen, daß die Farbtemperatur des emittierten Lichtes konstant gehalten werden kann.

In vorteilhafter Ausgestaltung der Erfindung ist vorgesehen, einen oder mehrere Photodetektoren zu verwenden, dessen/deren Spektralempfindlichkeit ein Erfassen von Licht auch auf Wellenlängen ermöglicht, die im NIR-Bereich oder im Infrarot-Bereich liegen. Eine solche Einrichtung hat den Vorteil, daß Fremdmaterial auch dann erfaßbar ist, wenn dessen Farbe mit der Basisfarbe des zu überprüfenden Garnes übereinstimmt.

Um negative Einflüsse auf das Meßergebnis durch Umgebungslicht auszuschließen, ist gemäß einer weiteren Ausführungsform vorgesehen, das von der Lichtquelle emittierte Licht zu modulieren.

Weitere vorteilhafte Ausgestaltungen der Erfindung sind den nachfolgend anhand der Zeichnungen erläuterten Ausführungsbeispielen entnehmbar. Es zeigen:
- Fig. 1: eine erste Ausführungsform der erfindungsgemäßen Fremdfasererkennungsvorrichtung,
- Fig. 2: ein weiteres Ausführungsbeispiel der erfindungsgemäßen Fremdfasererkennungsvorrichtung,
- Fig. 3: Einzelheiten der Fremdfasererkennungsvorrichtung, in vergrößertem Maßstab.

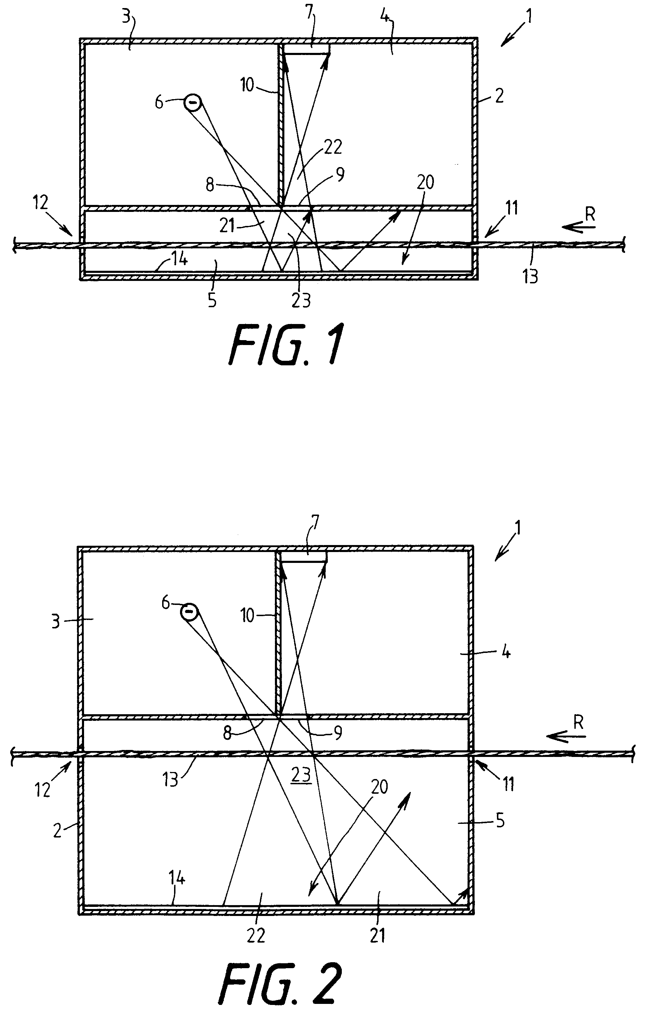

Die in den Fig. 1 und 2 schematisch dargestellte Fremdfasererkennungsvorrichtung 1 ist als optischer Detektor ausgebildet. Das Gehäuse 2 ist in drei Bereiche, einen Beleuchtungsteil 3, einen Empfangsteil 4 sowie einen Fadenabtastraum 5 unterteilt. Im Beleuchtungsteil 3 ist eine Lichtquelle 6, im Empfangsteil 4 eine Sensoreinrichtung 7 angeordnet. Der Faserabtastraum 5 weist an seiner dem Beleuchtungsteil und dem Empfangsteil gegenüberliegenden Wandung ein Spiegelelement 14 auf. Der Beleuchtungsteil 3 und der Empfangsteil 4 sind über eine lichtdichte Zwischenwand 10 getrennt und weisen in Richtung des Fadenabtastraumes 5 eine Lichtaustrittsöffnung 8 beziehungsweise eine Lichteintrittöffnung 9 auf. Die Lichtöffnungen 8, 9 sind vorzugsweise durch eine kratzfeste Glasplatte oder dergleichen optisch durchgängig verschlossen. Der Fadenabtastraum 5 wird von einem Faden 13 in Richtung R durchlaufen. Der Fadeneinlaß 11 beziehungsweise der Fadenauslaß 12 sind dabei vorzugsweise als schmale, lichtenge Schlitze ausgeführt.

Ein vom Licht der polychromaten Lichtquelle 6 ausgeleuchteter Bereich 21 und ein von der Sensoreinrichtung 7 erfaßbarer Bereich 22 bilden innerhalb des Fadenabtastraumes 5 einen gemeinsamen "Meßbereich" 23, der vom Faden 13 durchquert wird. Die Lichtquelle 6 ist vorzugsweise als miniaturisierte Kugellampe ausgeführt; das Spektrum des emittierten Lichtes erstreckt sich vom sichtbaren - bis in den nahen Infrarotbereich. Um Einflüsse durch Umgebungslicht, das eventuell durch die Fadendurchgangsöffnungen in den Fadenabtastraum eindringen kann, auszuschalten, kann außerdem eine Modulation des Lichtes vorgesehen werden.

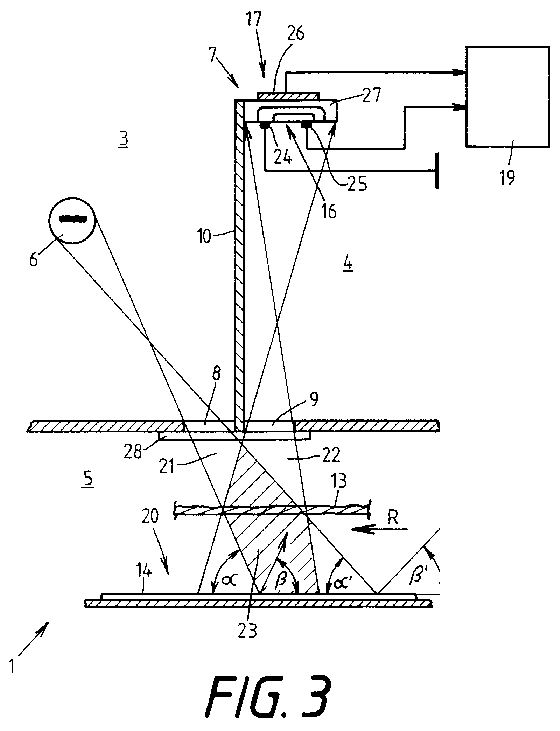

Als Sensoreinrichtung 7 findet ein Wellenlängen - selektiver Photoempfänger Verwendung. Die Sensoreinrichtung 7 weist dabei zwei Photodetektoren 16, 17 auf, die in einem gemeinsamen Si-Substrat vertikal gestaffelt angeordnet sind. Bei Strahlungseinfall erzeugen die beiden Photodetektoren, die, wie in Fig. 3 angedeutet, eine gemeinsame Kathode 24 und vertikal getrennte Anode 25, 26 aufweisen, zwei Photoströme. Aufgrund der unterschiedlichen vertikalen Lage gelten dabei für die Signale unterschiedliche spektrale Empfindlichkeiten, wobei entsprechend des Spektralabsorptionsvermögens des Basismaterials das Licht mit der kürzeren Wellenlänge von der Kopfdiode 16 aufgenommen wird, während längere Wellenlängen ein tieferes Eindringen der Photonen in das Si-Substrat erlauben und ein Signal an der Basisdiode 17 ergeben.

Die vom Sensorelement 7 erfaßbaren Wellenlängenbereiche hängen unter anderem vom Basismaterial ab, in das die Photodetektoren 16, 17 eingebettet sind. Ein einfaches Si-Substrat erlaubt beispielsweise eine Zerlegung des Lichtes in Wellenlängenbereiche zwischen 400 bis 1.000 nm. Bei Verwendung von Si-Ge, Si-PbS oder Si-PbSe kann dieser Bereich bis in den NIR-Bereich oder den Infrarot-Bereich ausgedehnt werden. In diesem Fall ist es durch die Verwendung von Photodioden mit der entsprechenden Spektralempfindlichkeit möglich, Fremdmaterial auch dann zu erfassen, wenn dessen Farbe mit der Basisfarbe des zu überprüfenden Garnes übereinstimmt.

Am Boden des Fadenabtastraumes 5 ist ein Spiegelelement 14 bezüglich der Beleuchtungseinrichtung (Lichtquelle 6, Lichtaustrittsöffnung 8) sowie bezüglich der Empfangseinrichtung (Lichteintrittsöffnung 9, Sensorelement 7) so angeordnet, daß lediglich der Teil des von der Lichtquelle 6 emittierten Lichtes von der Sensoreinrichtung erfaßbar ist, der vom Garn 13 direkt reflektiert wird, wenn das Garn 13 den Meßbereich 23 kreuzt. Das übrige Licht wird vom Spiegelelement 14 entsprechend der physikalischen Gesetzmäßigkeit Lichteinfallswinkel = Lichtausfallswinkel so reflektiert, daß es für das Sensorelement 7 unsichtbar ist. Auf diese Weise gelingt es, den Hintergrund auszuschalten und einen sogenannten "schwarzen Spiegel" zu schaffen.

Bei stark flusenden oder abriebsempfindlichen Garnen kann es eventuell zu einer Verschmutzung des Spiegelelementes 14 durch Staub oder dergleichen kommen, was zu einer diffusen Reflexion des von der Lichtquelle 6 emittierten Lichtes und damit zu negativen Auswirkungen auf die Meßergebnisse führen kann.

Solche negativen Auswirkungen werden durch eine Ausführungsform vermieden, wie sie in Fig. 2 dargestellt ist. Das Spiegelelement 14 ist hier relativ weit vom Beleuchtungsteil 3 und vom Empfangsteil 4 entfernt am unteren Ende des Meßbereiches 23 angeordnet, so daß sichergestellt ist, daß selbst bei diffusen Lichtreflexionen der Hintergrund nicht mehr vom Sensorelement 7 wahrnehmbar ist. Wie bereits vorstehend erwähnt, ist ein besonderer Vorteil dieser "Hintergrundentfernung", daß bezüglich der Basisfarbe des Garnes keinerlei Einschränkung mehr besteht und daher jederzeit und ohne besondere Maßnahmen ein Wechsel der Garnfarbe möglich ist.

### Funktion der Vorrichtung:

Die im Beleuchtungsteil 3 des Gehäuses 2 der Fremdfasererkennungsvorrichtung 1 angeordnete polychromate Lichtquelle 6 emittiert Licht, das durch eine Lichtaustrittsöffnung 8 in einen Fadenabtastraum 5 tritt und dort einen Bereich 21 ausleuchtet. Gleichzeitig wird von einem Photoempfänger 7 durch eine Lichteintrittsöffnung 9 hindurch ein Bereich 22 sensorisch überwacht. Der ausgeleuchtete Bereich 21 und der sensorisch erfaßbare Bereich 22 bilden einen gemeinsamen Meßbereich 23, der von dem zu überwachenden Faden 13 in Richtung R durchlaufen wird. Beim Kreuzen des Meßbereiches 23 wird von der Oberfläche des Fadens 13 ein Teil des von der Lichtquelle 6 auf verschiedenen Wellenlängen emittierten Lichtes reflektiert und diese Reflexion von den eine unterschiedliche Spektralempfindlichkeit aufweisenden Photodetektoren 16, 17 der Sensoreinrichtung 7 erfaßt.

Zu Beginn des Betriebes wird zunächst der "normale" Farbkoeffizient für eine vorbestimmte Garnlänge ermittelt. Danach wird an der nachgeschalteten Steuereinheit 19 die Verstärkung eines der von der Sensoreinrichtung 7 erfaßten Signale angepaßt, bis man einen Null-Ausgang erhält. Die Anwesenheit von Fremdfasern bewirkt ein Anwachsen oder Absinken dieses Ausgangssignales, was mittels eines Komparators registriert wird.

Gemäß der in Fig. 1 und 2 dargestellten Anordnung beleuchtet die Lichtquelle 6 den Meßbereich 23 aus einer Richtung, wobei die spektrale Zusammensetzung des Lichtes innerhalb dieses Bereiches gleichförmig ist. Das emittierte Licht weist dabei wenigstens auf zwei Wellenlängen eine analysierbare Intensität auf. Die Reflexion des Lichtes an der Oberfläche des Fadens 13 wird durch Wellenlängen - selektive Photodetektoren 16, 17, die in gestaffelter Anordnung in einer Basismasse, zum Beispiel einem Si-Substrat 27 des Sensorelementes 7 eingebettet sind, unter einem identischen Winkel erfaßt.

Da das von den Photodetektoren 16 beziehungsweise 17 erfaßbare Licht unterschiedlicher Wellenlängen den gleichen optischen Weg aufweist, das heißt, von derselben physikalischen Lichtquelle stammt und einer identischen Reflexion unterworfen ist, werden Einflüsse, die durch die Garnstruktur bedingt sind, weitestgehend ausgeschaltet. Garndurchmesserschwankungen, die zwar die Intensität des reflektierten Lichtes, nicht aber dessen Spektralverteilung beeinflussen und sich auf allen Wellenlängen identisch auswirken, werden dadurch ausgeschaltet, daß das Verhältnis beziehungsweise die Differenz der Logarithmen der auf unterschiedliche Wellenlängen basierenden Signale berücksichtigt wird.

Der Anteil des emittierten Lichtes, der nicht vom Faden 13 reflektiert wird, trifft auf ein am Boden des Fadenabtastraumes 5 angeordnetes Spiegelelement 14. Die geometrische Anordnung des Spiegelelemtes 14 in bezug auf den Beleuchtungsteil (Lichtquelle 6, Lichtaustrittsöffnung 8) sowie den Empfangsteil (Lichteintrittsöffnung 9, Photoempfänger 7) ist dabei so getroffen, daß diese Lichtkomponenten für das Sensorelement 7 unsichtbar bleiben. Auf diese Weise wird ein für das Sensorelement 7 nicht erfaßbarer Hintergrund 20, ein sogenannter "schwarzer Spiegel" geschaffen, der insbesondere den Vorteil hat, daß auch bei einem Wechsel der Farbe des zu überprüfenden Garnes 13 keine Anpassung des Hintergrundes notwendig wird.

Die in Fig. 2 dargestellte Ausführungsform zeigt einen Fadenabtastraum 5, dessen Spiegelelement 14 relativ weit von den Lichtöffnungen 8 und 9 entfernt, am unteren Ende des Meßbereiches 23 angeordnet ist. Mit einer solchen Anordnung läßt sich vermeiden, daß das Meßergebnis durch diffuse Lichtreflexionen, wie sie bei einer Verschmutzung des Spiegelelementes 14 auftreten können, verfälscht wird.

Die erfindungsgemäße Fremdfasererfassungsvorrichtung gewährleistet aufgrund der gewählten Auswahl der Einzelkomponenten sowie deren spezielle Anordnung, die Möglichkeit einer klaren Unterscheidung zwischen Grundmaterial und Fremdkörpern, wobei Garndurchmesserschwankungen oder Garnoberflächenstrukturänderungen die Meßergebnisse nicht beeinflussen. Die Vorrichtung ist außerdem unabhängig von der Grundfarbe des zu überprüfenden Garnes.

In alternativer Ausführungsform kann das erhaltene Meßergebnis außerdem mit einem bekannten Durchmessererfassungssensor kombiniert werden, so daß eine zuverlässigere und genauere Fehlererkennung und Reinigung des Garnes gewährleistet ist.

## Patentansprüche

1. Vorrichtung zum Erkennen von Fremdmaterial, insbesondere von Fremdfasern, in einem längsbewegten textilen Gebilde, mit einer Detektoreinrichtung, die wenigstens eine Licht emittierende Lichtquelle sowie eine Sensoreinrichtung zum Empfangen von reflektiertem Licht aufweist,
dadurch gekennzeichnet,
daß eine Lichtquelle (6) zum Emittieren von Licht wenigstens zweier verschiedener Wellenlängen und wenigstens zwei Photodetektoren (16, 17) unterschiedlicher Spektralempfindlichkeit zum Erfassen von reflektiertem Licht der wenigstens zwei verschiedenen Wellenlängen vorgesehen sind.

2. Vorrichtung nach Anspruch 1, dadurch gekennzeichnet, daß eine polychromate Lichtquelle (6) Verwendung findet und die Photodetektoren (16, 17) so angeordnet sind, daß das reflektierte, von den Photodetektoren (16, 17) empfangene Licht verschiedener Wellenlängen einen gleichen optischen Weg durchläuft.

3. Vorrichtung nach Anspruch 1 und 2, dadurch gekennzeichnet, daß die Anordnung der Photodetektoren (16, 17) und des optischen Weges so gewählt sind, daß das reflektierte Licht für beide Photodetektoren vom selben Meßort stammt und unter gleichem Winkel auf die Photodetektoren auftrifft.

4. Vorrichtung nach Anspruch 3, dadurch gekennzeichnet, daß zum Erfassen des reflektierten Lichtes verschiedener Wellenlängen vertikal gestaffelt angeordnete Photodioden (16, 17) vorgesehen sind.

5. Vorrichtung nach Anspruch 1, dadurch gekennzeichnet, daß Mittel zur Abschattung der Photodioden (16, 17) bezüglich der Lichtkomponenten vorgesehen sind, die nicht direkt vom textilen Gebilde (13) reflektiert werden.

6. Vorrichtung nach Anspruch 5, dadurch gekennzeichnet, daß als Mittel zur Abschattung der Photodioden (16, 17) ein unter Berücksichtigung der physikalischen Gesetzmäßigkeit Lichteinfallswinkel = Lichtausfallswinkel positioniertes Spiegelelement (14) Verwendung findet.

7. Vorrichtung nach Anspruch 5, dadurch gekennzeichnet, daß als Mittel zur Abschattung der Photodioden (16, 17) Blenden oder dergleichen Verwendung finden.

8. Vorrichtung nach einem oder mehreren der vorherigen Ansprüche, dadurch gekennzeichnet, daß ein Komparator Verwendung findet, der das Verhältnis der in den Photodioden (16, 17) initiierten Meßströme bildet.

9. Vorrichtung nach Anspruch 8, dadurch gekennzeichnet, daß die Differenz der Logarithmen der Meßströme gebildet wird.

10. Vorrichtung nach Anspruch 8 und 9, dadurch gekennzeichnet, daß als Logarithmusfunktion die Logarithmuscharakteristik der Basis-Emitter-Diode Anwendung findet.

11. Vorrichtung nach einem oder mehreren der vorherigen Ansprüche, dadurch gekennzeichnet,
- daß die polychromate Lichtquelle (6) und die als Wellenlängen - selektiver Photoempfänger mit wenigstens zwei, vertikal gestaffelt angeordneten Photodetektoren (16, 17) ausgebildete Sensoreinrichtung (7) in einem Gehäuse (2) der Detektoreinrichtung (1) so angeordnet sind, daß zwischen ihnen kein direkter optischer Kontakt gegeben ist, wobei ein Spiegelelement (14) vorgesehen ist, welches den Anteil des emittierten Lichtes, der nicht vom textilen Gebilde (13) reflektiert wird, so ablenkt, daß er für das Sensorelement (7) nicht erfaßbar ist.

12. Vorrichtung nach einem oder mehreren der vorherigen Ansprüche, dadurch gekennzeichnet, daß ein die Lichtquelle (6) aufnehmender Beleuchtungsteil (3) und ein die Sensoreinrichtung (7) aufnehmender Empfangsteil (4) durch eine lichtdichte Abschirmung (10) getrennt sind und eine Lichtaustrittsöffnung (8) beziehungsweise eine Lichtaustrittsöffnung (9) aufweisen, wobei im Abstand vor diesen Öffnungen (8, 9) das Spiegelelement (14) so angeordnet ist, daß ein vom Sensorelement (7) nicht erfaßbarer "schwarzer Spiegel" (20) entsteht.

13. Vorrichtung nach einem oder mehreren der vorherigen Ansprüche, dadurch gekennzeichnet, daß ein von der Lichtquelle (6) ausgeleuchteter Bereich (21) und ein von der Sensoreinrichtung (7) detektierbarer Bereich (22) einen gemeinsamen Meßbereich (23) bilden, der, zwischen den Lichtöffnungen (8, 9) und dem Spiegelelement (14) angeordnet, von einem textilen Gebilde, vorzugsweise einem Faden (13), durchlaufen wird.

14. Vorrichtung nach einem oder mehreren der vorherigen Ansprüche, dadurch gekennzeichnet, daß die geometrische Anordnung von Beleuchtungseinrichtung (Lichtquelle (6), Lichtaustrittsöffnung (8)), Empfangseinrichtung (Lichteintrittsöffnung (9), Photoempfänger (7)) und Spiegelelement (14) in der Weise getroffen ist, daß der Photoempfänger (7) nur mit dem Teil des von der Lichtquelle (6) emittierten Lichtes beaufschlagt wird, der von der Oberfläche des den Meßbereich (23) kreuzenden Fadens (13) direkt reflektiert wird.

15. Vorrichtung nach einem oder mehreren der vorherigen Ansprüche, dadurch gekennzeichnet, daß der den Meßbereich (23) kreuzende Faden (13) parallel zum Spiegelelement (14) verläuft.

16. Vorrichtung nach einem oder mehreren der vorherigen Ansprüche, dadurch gekennzeichnet, daß die geometrische Anordnung von Beleuchtungseinrichtung (6, 8), Empfangseinrichtung (9, 7) und Spiegelelement (14) in der Weise getroffen ist, daß eine Beaufschlagung des Photoempfängers (7) durch vom Spiegelelement (14) reflektiertem Streulicht vermieden wird (Fig. 2).

17. Vorrichtung nach einem oder mehreren der vorherigen Ansprüche, dadurch gekennzeichnet, daß das von der polychromaten Lichtquelle (6) emittierte Licht ein Spektrum aufweist, das sich vom sichtbaren - bis in den NIR-Bereich erstreckt.

18. Vorrichtung nach Anspruch 17, dadurch gekennzeichnet, daß das von der polychromaten Lichtquelle (6) emittierte Licht wenigstens auf zwei Wellenlängen eine von den Photodetektoren (16, 17) erfaßbare Intensität aufweist.

19. Vorrichtung nach einem oder mehreren der vorherigen Ansprüche, dadurch gekennzeichnet, daß wenigstens einer der Photodetektoren eine Spektralempfindlichkeit aufweist, die ein Erfassen von Licht auf Wellenlängen ermöglicht, die im NIR-Bereich oder im Infrarot-Bereich liegen.

20. Vorrichtung nach Anspruch 17 und 18, dadurch gekennzeichnet, daß eine Lichtquelle (6) Verwendung findet, die ein Konstanthalten der Farbtemperatur des Lichtes ermöglicht.

21. Vorrichtung nach einem oder mehreren der vorherigen Ansprüche, dadurch gekennzeichnet, daß das von der Lichtquelle (6) emittierte Licht modulierbar ist.

22. Vorrichtung nach Anspruch 11, dadurch gekennzeichnet, daß die Sensoreinrichtung (7) wenigstens zwei, in ein Basissubstrat (27) eingebettete, vertikal versetzt angeordnete Photodioden (16, 17) aufweist, wobei die im Bereich des Sensorkopfes angeordnete Photodiode (16) das kurzwelligere und die am Sensorfuß angeordnete Photodiode (17) das langwelligere Licht verarbeitet.

23. Vorrichtung nach Anspruch 12, dadurch gekennzeichnet, daß die Lichtaustrittsöffnung (8) und die Lichteintrittöffnung (9) durch eine kratzfeste Glasplatte (28) abgedeckt sind.

24. Vorrichtung nach Anspruch 12, dadurch gekennzeichnet, daß im Bereich der Lichteintrittsöffnung (9) und/oder der Lichtaustrittsöffnung (8) ein spezielle Garneigenschaften erfaßendes optisches Mittel, beispielsweise ein Farbfilter, ein Polarisationsfilter etc., angeordnet ist.
